# EUROPEAN PATENT SPECIFICATION

(11) **EP 3 191 509 B1**
(45) Date of publication and mention of the grant of the patent: **20.02.2019**
(21) Application number: 15760451.3
(22) Date of filing: 08.09.2015
(51) Int. Cl.: C07K 16/18

(54) **ANTIBODIES SPECIFIC FOR COMPLEMENT COMPONENT C4D AND USES THEREOF**
KOMPLEMENTKOMPONENTE C4D-SPEZIFISCHE ANTIKÖRPER UND VERWENDUNGEN DAVON
ANTICORPS SPÉCIFIQUES DE COMPOSANT COMPLÉMENT C4D ET LEURS UTILISATIONS

(30) Priority: 09.09.2014 EP 14184140; 27.01.2015 EP 15152765
(43) Date of publication of application: 19.07.2017
(73) Proprietor: Euro-Diagnostica AB, 202 11 Malmö (SE)
(72) Inventor: BLOM, Anna, S-20502 Malmö (SE); OKRÓJ, Marcin Sebastian, S-20502 Malmö (SE)
(74) Representative: Zacco Denmark A/S
(86) International application number: PCT/EP2015/070526
(87) International publication number: WO 2016/038055

(56) References cited:
- US-A- 5 221 616
- ALI S A ET AL: "Frequent expression of C4d in hepatic graft-versus-host disease: Potential clue for diagnosis and distinguishing acute and chronic form", TRANSPLANT IMMUNOLOGY, ELSEVIER, NL, vol. 23, no. 1-2, 1 May 2010 (2010-05-01), pages 77-80, XP027089341, ISSN: 0966-3274 [retrieved on 2010-03-15] -& Anonymous: "Anti-C4d Antibody, Human, 12-5000 | ARP American Research Products, Inc.", , 1 January 2008 (2008-01-01), XP055152495, Retrieved from the Internet: URL:http://www.arp1.com/Anti-Human-C4d-Ant ibody-p/12-5000.htm [retrieved on 2014-11-12]
- Anonymous: "Anti-C4d antibody, prediluted ab58781, abcam", , 1 January 2014 (2014-01-01), XP055152498, Retrieved from the Internet: URL:http://www.abcam.com/C4d-antibody-pred iluted-ab58781.pdf [retrieved on 2014-11-12]
- Anonymous: "Polyclonal Antibody to Complement C4d (C-term) - Purified, Acris Antibodies, Inc.", , 1 January 2013 (2013-01-01), XP055152500, Retrieved from the Internet: URL:http://m1.acris-antibodies.com/pdf/AP1 5376PU-S.pdf [retrieved on 2014-11-12]
- Shinji Maeda ET AL: "Identification of a surface structure in the fourth component of human complement, C4, which becomes hidden upon activation by Cis", Biochem. J, 1 January 1993 (1993-01-01), pages 503-508, XP055152434, Retrieved from the Internet: URL:http://www.biochemj.org/bj/289/0503/28 90503.pdf [retrieved on 2014-11-12]
- BERGSETH GRETHE ET AL: "An international serum standard for application in assays to detect human complement activation products", MOLECULAR IMMUNOLOGY, vol. 56, no. 3, 17 June 2013 (2013-06-17), pages 232-239, XP028683649, ISSN: 0161-5890, DOI: 10.1016/J.MOLIMM.2013.05.221
- BLOM ANNA M ET AL: "Antibodies reactive to cleaved sites in complement proteins enable highly specific measurement of soluble markers of complement activation", MOLECULAR IMMUNOLOGY, vol. 66, no. 2, 18 March 2015 (2015-03-18) , pages 164-170, XP029170616, ISSN: 0161-5890, DOI: 10.1016/J.MOLIMM.2015.02.029

## Description

### Field of the invention

The invention is in the field of medical diagnostics and other medical tools, as well as uses thereof. More in particular, it provides antibodies specifically directed against a component of the complement system, which allows an improved determination of the activation of the classical complement pathway. Even more in particular, the invention provides means and methods for detecting the activation of the classical complement pathway. Furthermore, the invention provides means and methods for determining the presence of complement component C4d in a biological sample.

### Background of the invention

The complement system helps or "complements" the ability of antibodies and phagocytic cells to clear pathogens from an organism. It is part of the immune system called the innate immune system [1] that is not adaptable and does not change over the course of an individual's lifetime. However, it can be rapidly recruited and brought into action by the adaptive immune system.

The complement system consists of a number of proteins found in the blood, in general synthesized by the liver, and normally circulating as inactive precursors (pro-proteins). When stimulated by one of several triggers, proteases in the system cleave specific proteins to release anaphylatoxins and initiate an amplifying cascade of further cleavages. The end-result of this activation cascade is massive amplification of the response and activation of the cell-killing membrane attack complex. Over 30 proteins and protein fragments make up the complement system, including soluble proteins found at high concentrations in blood and cell membrane receptors. They account for about 5% of the globulin fraction of blood serum and can serve as opsonins.

The proteins that constitute the complement system are synthesized mainly by hepatocytes. Significant amounts are also produced by tissue macrophages, blood monocytes, and epithelial cells of the genitourinary tract and gastrointestinal tract. The complement system can be initiated by three biochemical pathways: the classical pathway, the alternative pathway, and the lectin pathway [2].

The three pathways of activation all generate analogous proteolytic C3-convertases and the common terminal pathway resulting in membrane attack formation. The classical complement pathway typically requires antigen:antibody complexes (immune complexes) for activation, whereas the alternative and mannose-binding lectin pathways can be activated by spontaneous C3 hydrolysis or carbohydrates without the presence of antibodies, respectively. In all three pathways, C3-convertase cleaves and activates component C3, creating C3a and C3b, and causing a cascade of further cleavage and activation events.

The classical pathway is triggered by activation of the C1-complex. The C1-complex is composed of one molecule of C1q, two molecules of C1r and two molecules of C1s, or C1qr²s². The C1 complex is activated when C1q binds to IgM or IgG complexed with antigens. A single IgM can initiate the pathway, while multiple IgGs are needed. This also occurs when C1q binds directly to the surface of the pathogen. Such binding leads to conformational changes in the C1q molecule, which leads to the activation of two C1r molecules. C1r is a serine protease. Activated C1r cleaves C1s (another serine protease). The C1r²s² component now splits C4 and then C2, producing C4a, C4b, C2a, and C2b. C4b and C2a bind to form the classical pathway C3-convertase (C4b2a complex), which promotes cleavage of C3 into C3a and C3b; C3b later joins with C4b2a (the C3 convertase) to make C5 convertase (C4b2a3b complex).

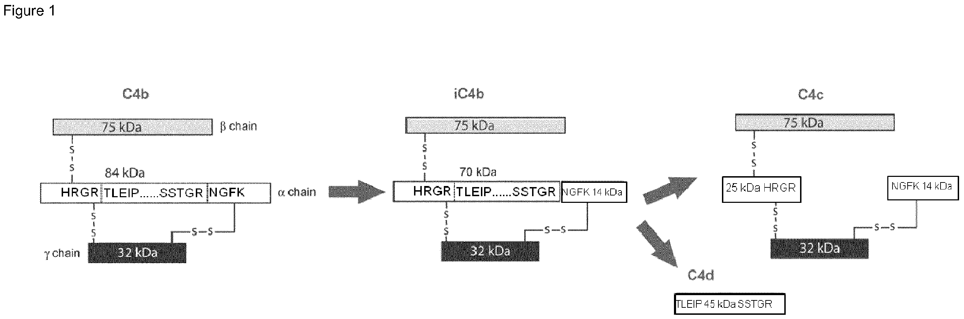

C4b formed during activation of the classical pathway is then quickly degraded by a serine protease factor I, which in the presence of a cofactor such as C4b-binding protein, generates C4d - the end product of C4b degradation, appearing only upon activation of the classical complement pathway. C4d is therefore an excellent marker for classical complement activation in vivo and in vitro. Recently, C4d in systemic blood was described as a diagnostic and prognostic marker of lung cancer [3]. It has been widely used as a marker of systemic lupus erythematosus (SLE) [4] and antibody-mediated graft rejection [5]. Although most of the C4d remains bound to the surface of the cell, which originally activated the classical complement pathway, certain portion is released and detectable in blood. The scheme of C4d formation is presented in figure 1.

In order to study and understand the role of C4d and the classical pathway activation underlying some diseases there is a need in the art for reagents, typically antibodies, which are specific for C4d. The present application addresses that need.

### Summary of the invention

The invention relates to an antibody specifically reactive with complement component C4d and not with complement component C4b or C4 wherein the antibody reacts with a peptide comprising an epitope consisting of amino acid sequence NVTLSSTGR-COOH and not with a peptide comprising an amino acid sequence NVTLSSTGRNGFK-COOH.

The invention also provides a method for generating such an antibody wherein a peptide essentially consisting of the amino acid sequence SSTGR-COOH is used as an immunogen.

The invention also provides a method for the detection of complement component C4d in a sample wherein the sample is contacted with an antibody as described herein and wherein binding between the antibody and C4d is detected.

The invention also provides a method for the diagnosis or prognosis of diseases selected from the group consisting of lung cancer, SLE, vasculitis and antibody-mediated graft rejection, wherein an antibody as described herein is contacted with a sample from an individual and wherein it is concluded that the individual has an increased risk of having or developing said disease if the sample contains an elevated level of complement component C4d in comparison with a sample of a control subject.

The invention also provides a method for monitoring disease activity wherein the disease is characterized by flares of disease activity. Such disease may be selected from the group consisting of SLE, vasculitis and antibody-mediated graft rejection, wherein an antibody as described herein is contacted with a sample from an individual and wherein the level of C4d present in the sample is monitored over time.

The invention also provides a method for determining the efficacy of the treatment of a disease. In particular if the disease is selected from the group consisting of lung cancer, SLE, vasculitis and antibody-mediated graft rejection. In such a method, the antibody as described herein is contacted with a sample taken from an individual before and after treatment and it is concluded that the individual responds to the treatment if the sample taken after treatment has a decreased or lower level of C4d in comparison to the C4d level in the sample taken before treatment.

### Detailed description of the invention

Antibodies reactive with C4d are known in the art and commercially available. However, in order to be useful as a diagnostic reagent for the presence of C4d, it is imperative that the antibody is specific for free C4d generated as result of complement activation, that means that it recognizes only C4d and not C4b or C4. Since C4d is a fragment of C4b and C4 this is not a trivial task.

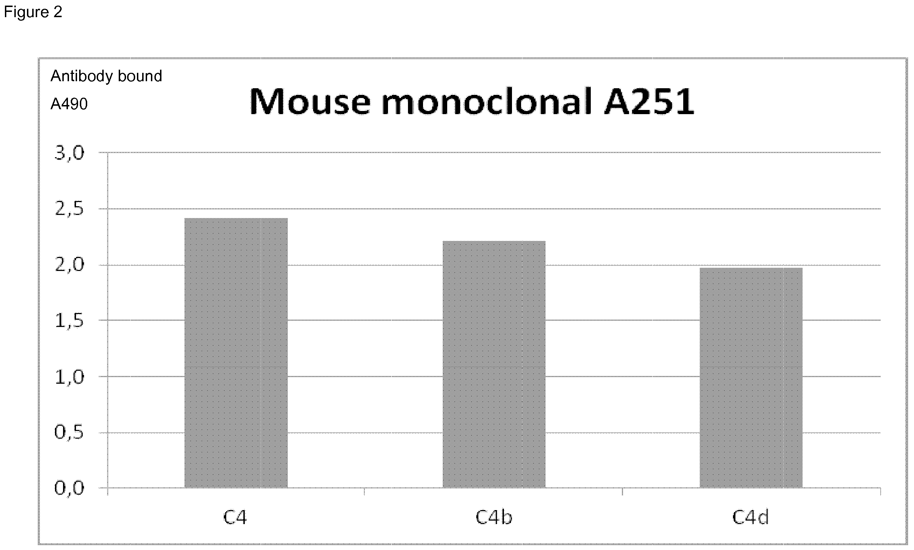

It has been reasoned [6-8] that activated complement proteins would express certain conformational epitopes, not present in their parental molecules. Such epitopes are often referred to as neo-epitopes. Antibodies against neo-epitopes in C4d are commercially available from Quidel Corporation (12544 High Bluff Drive, Suite 200, San Diego, CA 92130, USA, such as the A251 antibody, which is the basis for the commercially available MicroVue™ C4d EIA kit, marketed by Quidel Specialty Products. The Quidel C4d MicroVue ELISA is an assay recommended by International Complement Society as a test for measurement of C4d fragment in serum or plasma [9]. This assay is based on the monoclonal anti C4d antibody A251 as a catching antibody and employs a goat polyclonal anti-C4d as detecting antibody. We tested the A251 antibody for specific reactivity with C4d and found that it also recognizes C4 and C4b (Example 7, figure 2).

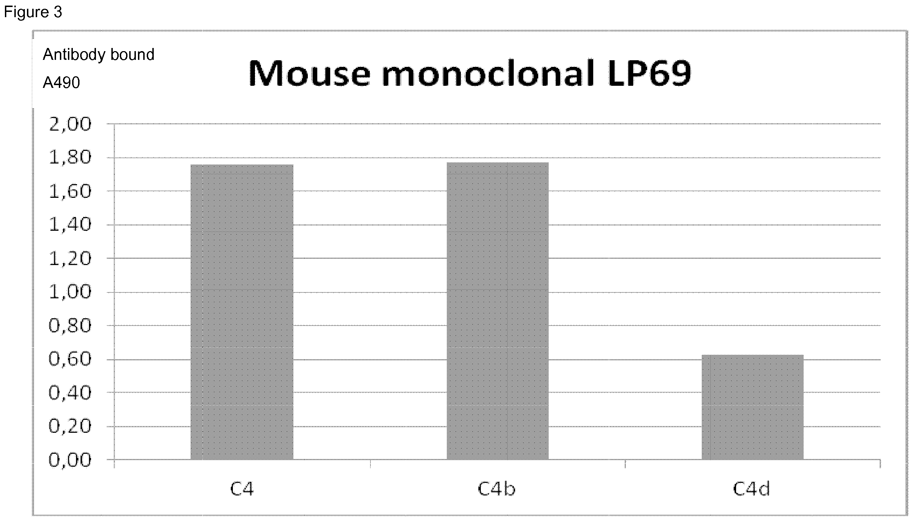

Another representative example of antibodies against C4d is the antibody Ab167093 (clone LP69) raised against C4d purified from human plasma. This antibody is commercially available from Abcam (330 Cambridge Science Park, Cambridge, CB4 0FL, UK). We tested that antibody for specificity towards C4d and it also appeared to cross-react with C4 and C4b (Example 7, figure 3). It therefore appears that C4d purified from human plasma is not a suitable source for generating C4d-specific antibodies.

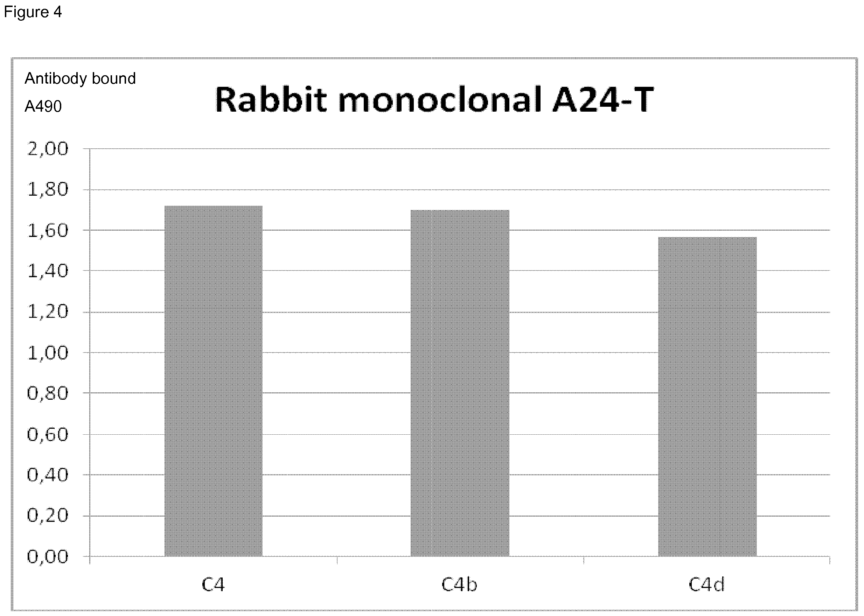

Another approach for raising C4d-specific antibodies relies on using synthetic peptides from an internal sequence of C4d as an immunogen. A representative example of this group is the rabbit monoclonal antibody Ab136921, clone A24-T, also commercially available from Abcam. We tested the Ab136921 antibody for specificity towards C4d and also this antibody appeared to cross-react with C4 and C4b (Example 7, figure 4).

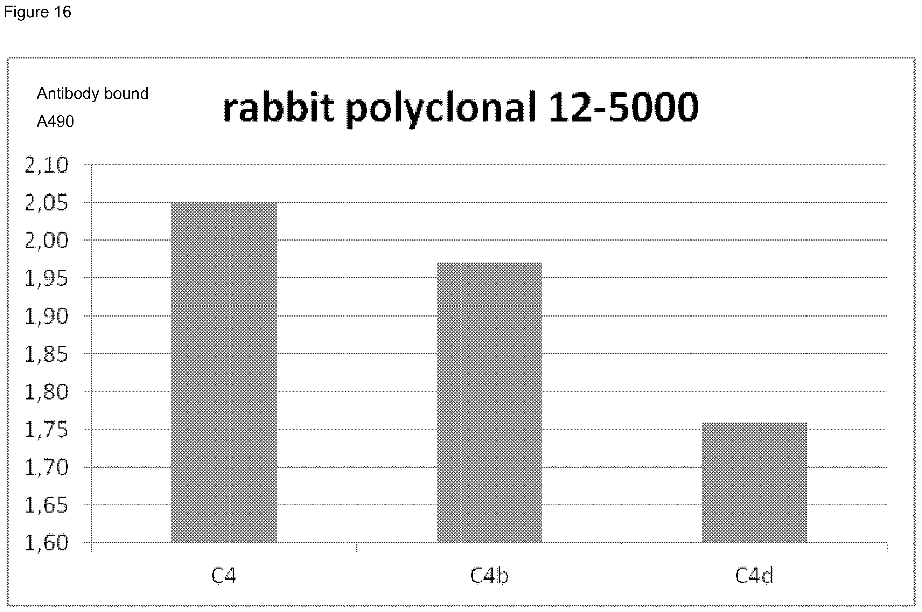

Another antibody allegedly specifically reactive with C4d is a rabbit polyclonal antibody 12-5000 described in reference No. 20 (Ali et al.). This antibody was obtained from American Research products and tested for specificity towards C4d. Also this antibody appeared to cross-react with C4 and C4b (Example 7, figure 16).

It therefore appears that internal peptides from C4d may also not be used as a suitable immunogen for obtaining C4d-specific antibodies.

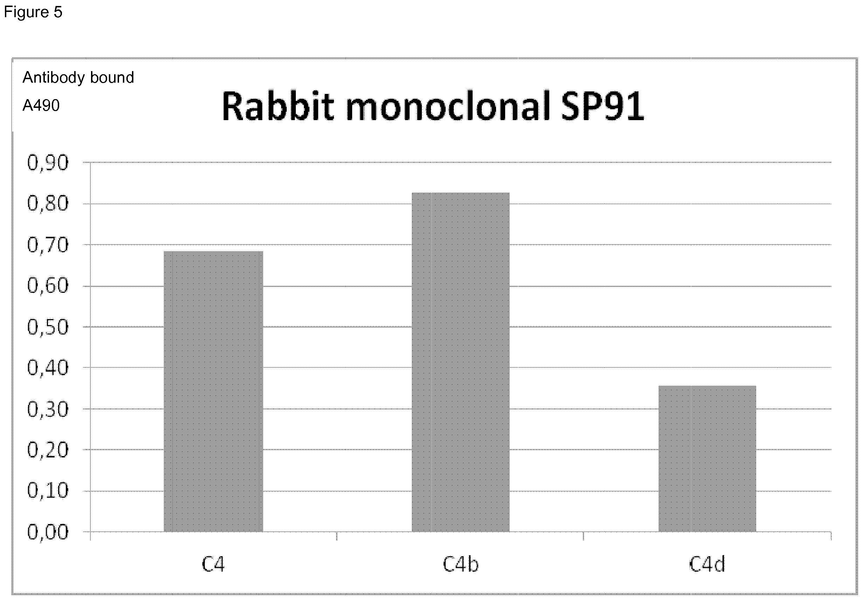

A further category of alleged C4d-specific antibodies are antibodies raised against a peptide from the C-terminal half of C4d. We tested three representative examples of such antibodies. Rabbit monoclonal antibody 183311, clone SP91, commercially available from Abcam, was found to be not specific for C4d either, since it reacts with C4 as well as C4b (figure 5).

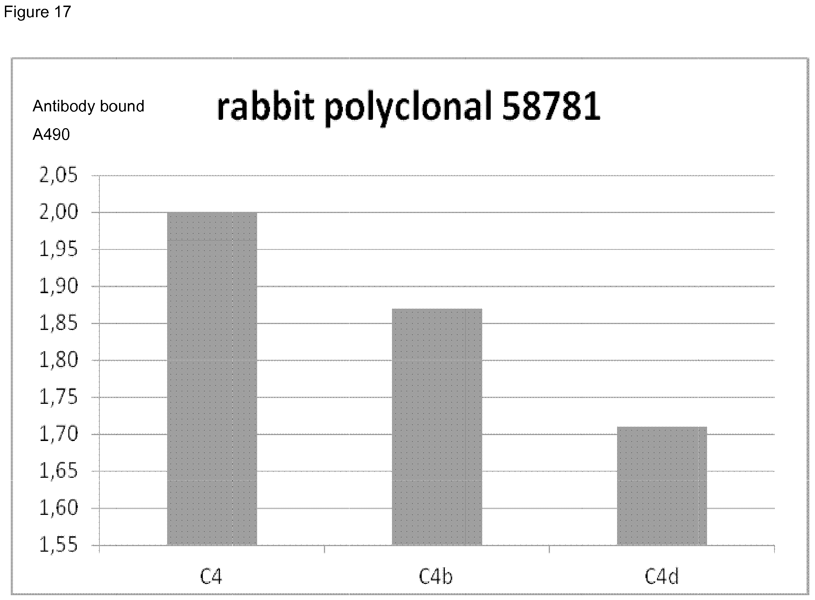

We also tested a polyclonal antibody from a rabbit immunized with a C-terminal peptide from C4d. This antibody (Ab 58781 commercially available from Abcam) is also not specific for C4d, since it reacts with C4 as well as C4b (figure 17).

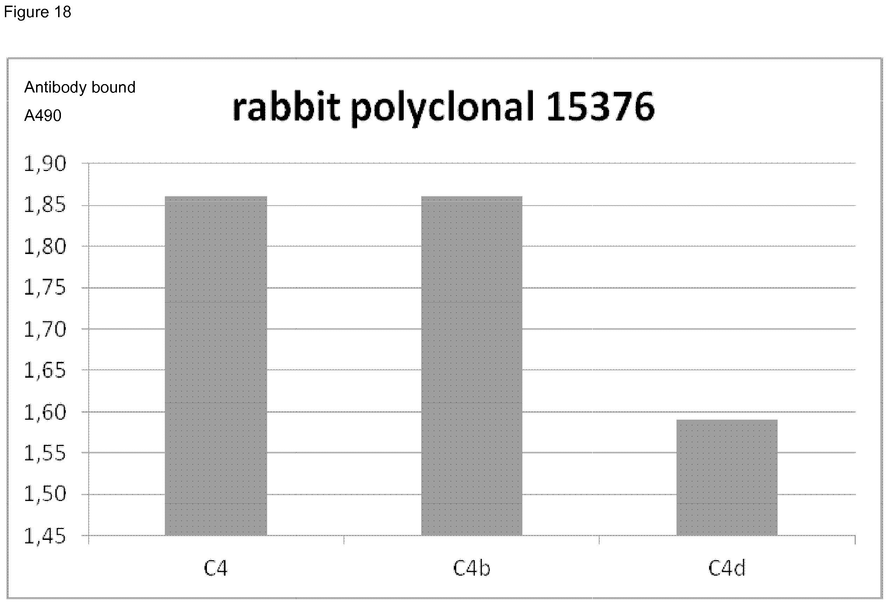

We also tested another polyclonal antibody from a rabbit immunized with a C-terminal peptide from C4d. This antibody (CPAMD2, catalogue number AP15376PU-S, commercially available from Acris) is also not specific for C4d, since it reacts with C4 as well as C4b (figure 18). These results show that immunization with a C-terminal peptide derived from C4d is also not an effective strategy for obtaining antibodies specific for C4d, which do not cross-react with C4b and/or C4.

In order to obtain truly specific antibodies against C4d, we immunized rabbits with peptides (two rabbits per peptide) corresponding to 5 amino acids from N-terminal and C-terminal sequences of C4d, attached to a glycine / cysteine spacer. This is detailed in Example 4. The sequence of the two peptides used for immunization was:
N-terminal C4d part: **NH2-TLEIPG-**GGC (SEQ ID NO: 1)
C-terminal C4d part: CGG**-SSTGR-COOH** (SEQ ID NO: 2)

The amino acids shown in bold typeface are part of the C4d amino acid sequence, the glycine and cysteine residues are used as spacers. Rabbits #1 and #2 were immunized with a peptide according to SEQ ID NO: 1. Rabbits #3 and #4 were immunized with a peptide according to SEQ ID NO: 2. Immunization and bleeding was performed by Agrisera AB (Sweden) and collected antiserum was tested in ELISA for reactivity with C4, C4b and C4d in the same assays as used above.

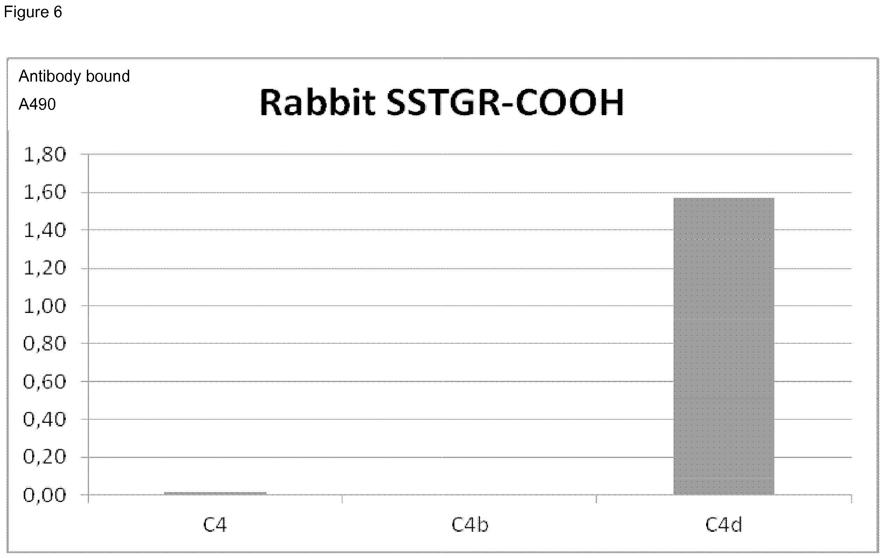

Both rabbits #3 and #4, immunized with the C-terminal peptide, comprising the sequence SSTGR-COOH yielded antibodies specific for C4d. They both reacted equally well with C4d and not with C4 or C4b (only antibodies from rabbit #3 are shown in figure 6). The two sera from rabbits #3 and #4 immunized with the C-terminal peptide CGGSSTGR-COOH (SEQ ID NO: 2) appeared to have identical specificity. Experiments described herein were all performed with antibodies from rabbit #3, when antibodies from rabbit #4 were used, they provided identical results. Antibodies from rabbit #3 are herein further referred to as antibody BLOK C4d. Antibodies from the two rabbits (#1 and #2) immunized with the N-terminal peptide comprising the sequence NH2-TLEIPG did not react with C4, C4b or C4d and were discarded.

The epitope recognized by BLOK C4d was then mapped to the extreme C-terminal end of C4d, as evidenced by the reactivity of BLOK C4d with the peptide Cys-NVTLSSTGR-COOH (SEQ ID NO: 3) and the absence of reactivity with the peptide Cys-NVTLSSTGRNGFK-COOH (SEQ ID NO: 4).

SEQ ID NO: 3 represents the extreme C-terminal end of C4d with the free carboxyl group, whereas SEQ ID NO: 4 represents the junction between the C-terminal end of C4d and C4c (see figure 1). It is therefore concluded that the BLOK C4d antibody recognizes an epitope specific for C4d, present at the extreme C-terminal end of C4d, which is not present in its precursor C4b or C4.

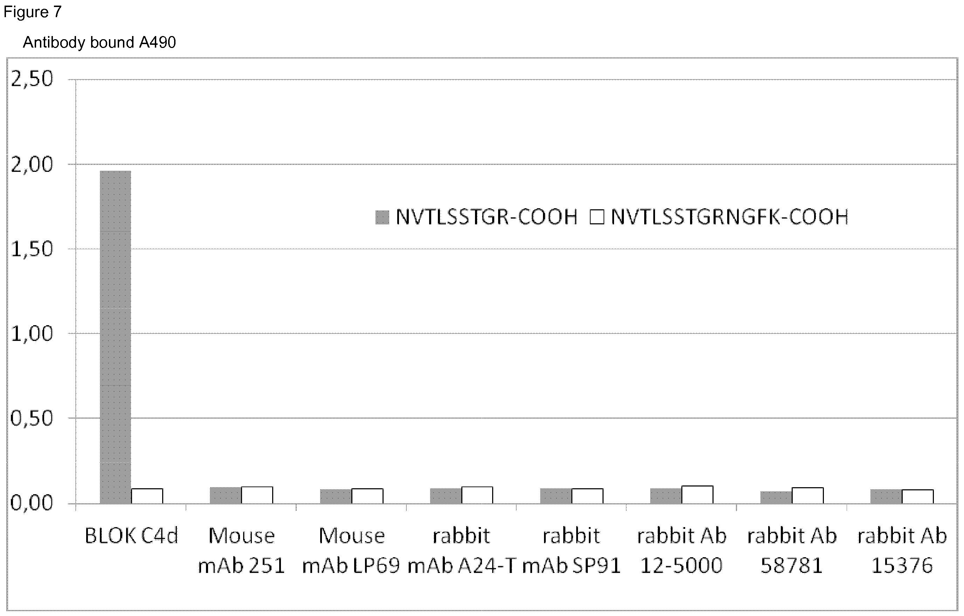

The above described commercially available anti-C4d antibodies were also tested for their reactivity with the peptides according to SEQ ID NO: 3 and SEQ ID NO: 4. None of the commercially available antibodies were reactive with peptides according to SEQ ID NO: 3 or SEQ ID NO: 4. This is graphically presented in figure 7.

The invention therefore relates in one aspect to an antibody specifically reactive with a peptide comprising an epitope consisting of amino acid sequence NVTLSSTGR-COOH and not with a peptide comprising an amino acid sequence NVTLSSTGRNGFK-COOH. Such antibodies are specifically reactive with C4d and not with C4 or C4b.

The expression "an antibody specifically reactive with a peptide comprising an epitope consisting of amino acid sequence NVTLSSTGR-COOH and not with a peptide comprising an amino acid sequence NVTLSSTGRNGFK-COOH" is used herein to indicate that the antibody is specifically reactive with an epitope at the C-terminus of C4d which comprises the amino acids SSTGR and a free C-terminal arginine residue (indicated as R-COOH). Moreover, the expression describes an antibody specifically reactive with a peptide comprising an epitope consisting of amino acid sequence NVTLSSTGR-COOH and not with the same peptide only differing by an extra stretch of 4 C-terminal amino acids (NGFK), i.e. the same peptide comprising an amino acid sequence NVTLSSTGRNGFK-COOH.

In yet again different terms, the antibody according to the invention may be described as an antibody reactive with an epitope comprised in peptide NVTLSSTGR-COOH and not in NVTLSSGRNGFK-COOH.

In another aspect, the invention relates to an antibody specifically reactive with complement component C4d and not with complement component C4b wherein the antibody reacts with a peptide comprising an epitope consisting of amino acid sequence NVTLSSTGR-COOH and not with a peptide comprising an amino acid sequence NVTLSSTGRNGFK-COOH.

Such antibodies may now be prepared employing conventional immunization methods known in the art. In a preferred embodiment, such antibodies are polyclonal antibodies. Polyclonal antibodies are known for high avidity and high affinity binding to their target antigens. In another preferred embodiment, an antibody according to the invention may be a monoclonal antibody, such as a mouse, rat or a rabbit monoclonal antibody. Such monoclonal antibodies have the advantage that they can be produced in an unlimited supply while maintaining the quality of the antibody.

Antibodies according to the invention may be obtained by conventional methods known in the art wherein a peptide essentially consisting of the amino acid sequence SSTGR-COOH is used as an immunogen. In other words, the invention relates to a method for generating an antibody as described above, wherein a peptide essentially consisting of the amino acid sequence SSTGR-COOH is used as an immunogen. The invention in another aspect therefore relates to an antibody obtainable by such a method.

The antibodies thus generated had considerable advantages over prior art antibodies and exhibited some unexpected properties as will be described herein.

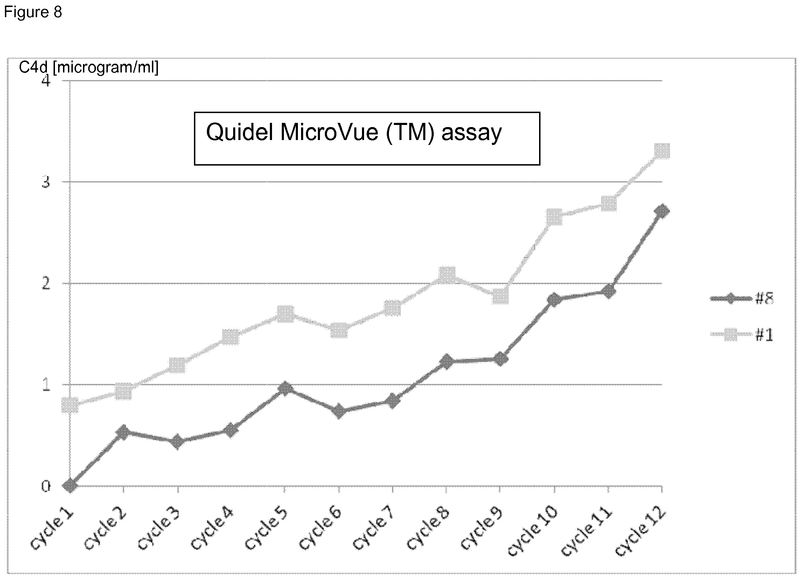

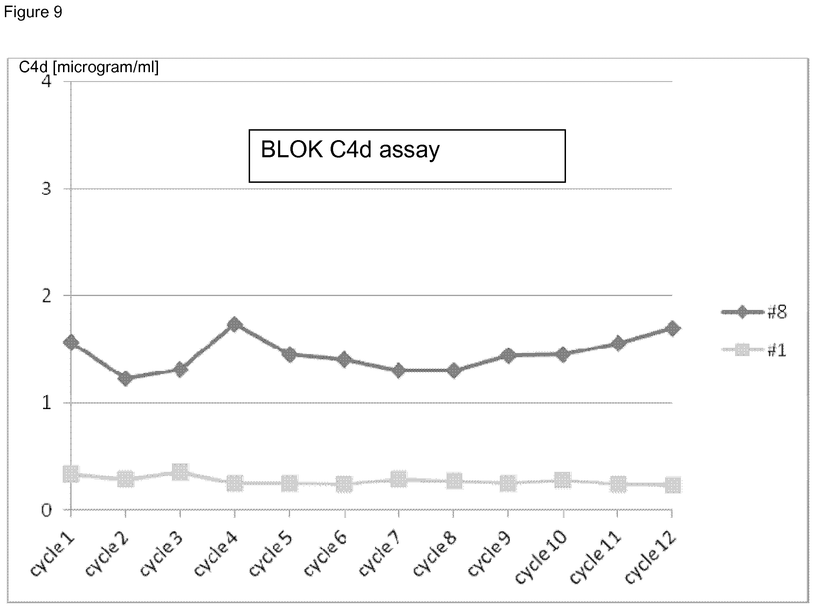

The Quidel MicroVue™ C4d EIA kit (cat. A008) wherein the A251 antibody is used as the capture antibody, is recommended by International Complement Society as a test for measurement of C4d fragment in serum or plasma [6]. We found that this assay suffers from a number of flaws and drawbacks. First of all, it provides inconsistent results when performed with samples that are frozen and thawed for a number of times. We show herein the results obtained with two samples (#1 and #8) that were thawed and frozen for 12 times. These samples showed C4d levels that increased with an increasing number of freeze-thaw cycles (figure 8). When these same samples were analyzed in a sandwich assay wherein the BLOK C4d antibody was used as a capture antibody, the results were consistent and did not vary with increasing number of freeze-thaw cycles (figure 9).

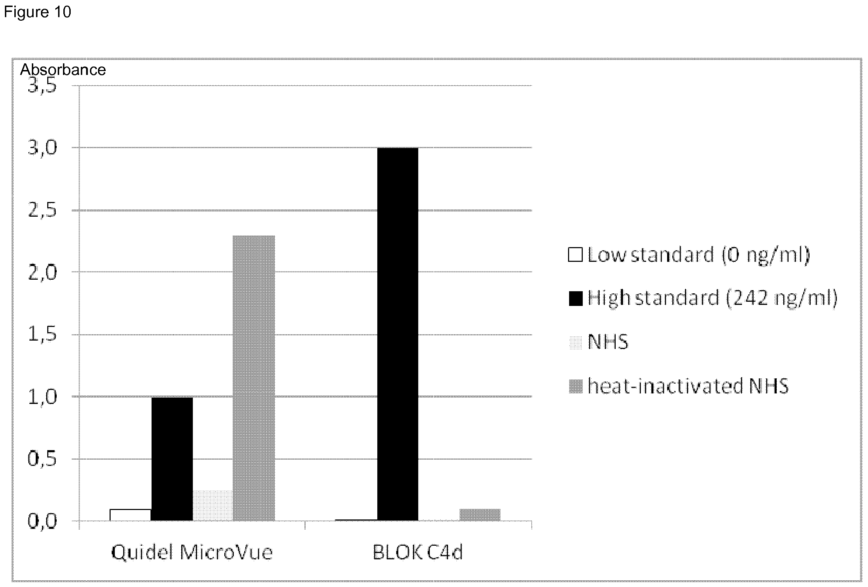

Another drawback of the commercially available Quidel MicroVue™ C4d EIA kit is that heat inactivated sera cannot be used. When a normal human serum (NHS) was tested before and after heat-inactivation, the Quidel assay caused a huge false-positive reaction, whereas the sandwich assay with the BLOK C4d antibody did not (figure 10).

The data presented in figure 10 also show that the assay employing the BLOK C4d antibody had a much higher and therefore better signal to noise ratio; whereas the Quidel MicroVue™ assay yielded a signal to noise ratio of 10 (high standard/low standard), the sandwich ELISA assay employing BLOK C4d as a capture antibody yielded a signal to noise ratio of 300 when the same high and low standards were used.

The antibodies according to the invention allowed therefore the production of an improved assay for the detection of C4d. The invention therefore also relates to an in vitro method for the detection of complement component C4d in a sample wherein the sample is contacted with an antibody as described above and wherein binding between the antibody and C4d is detected.

In an advantageous embodiment, the invention relates to a method as described above, wherein the sample is a blood, serum, plasma, synovial fluid or liquor sample. The method according to the invention may provide particularly good results when the sample is heat-inactivated or thawed several times as is often the case with archival samples from clinical studies.

The antibody according to the invention may be used in a number of immunological assay formats. Its use in ELISA, RIA, EIA, and immunohistochemistry is particularly preferred.

Furthermore, we undertook an evaluation of the activation of the classical complement pathway in leukemia patients undergoing treatment with Rituximab. Rituximab, when bound to cancer cells carrying CD20, binds C1 complex and activates directly the classical pathway.

We measured the level of C4d in samples obtained before and after the infusion with Rituximab and we also determined the level of Terminal Complement Complex (TCC), a more downstream marker of activation of the classical pathway.

TCC is generated as follows. C3-convertase activates C3 generating C3b, which then binds to the C3-convertase changing its specificity to become C5-convertase. This enzymatic complex activates C5 to C5b, which interacts with C6, C7, C8 and C9 to form TCC, which inserts into cell membranes causing cell death. Some of the C5b-9 complexes remain in the solution and can be measured by ELISA [10].

When a molecule such as Rituximab activates the classical pathway one would expect correlation between generated C4d (early in the pathway) and TCC (end of the pathway).

Plasma samples were obtained from non-Hodgkin's lymphoma or chronic lymphocytic leukemia (CLL) patients from Karolinska University Hospital in Stockholm, who were treated with anti-CD20 mAb (Rituximab™). Samples were collected only from patients naive for Rituximab. We obtained samples from 11 patients collected just before and after infusion with Rituximab and tested them in a C4d ELISA according to the invention employing the BLOK C4d antibody, and in Quidel's C4d MicroVue™ assay. The increase in the C4d level in the samples before and after Rituximab treatment was determined for both assays and compared with the level of Terminal Complement Complex (TCC).

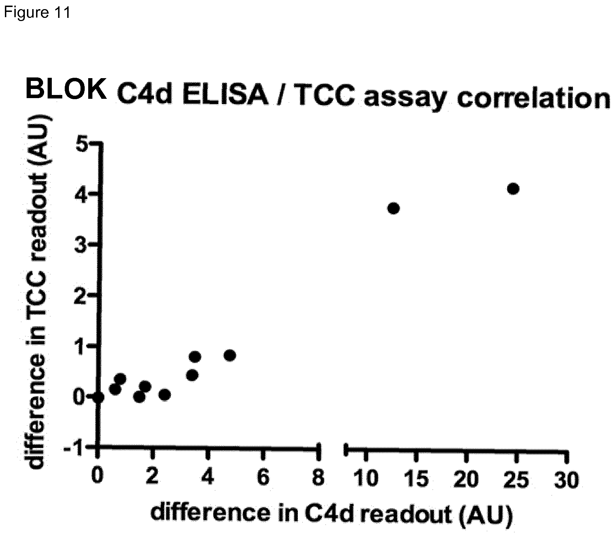

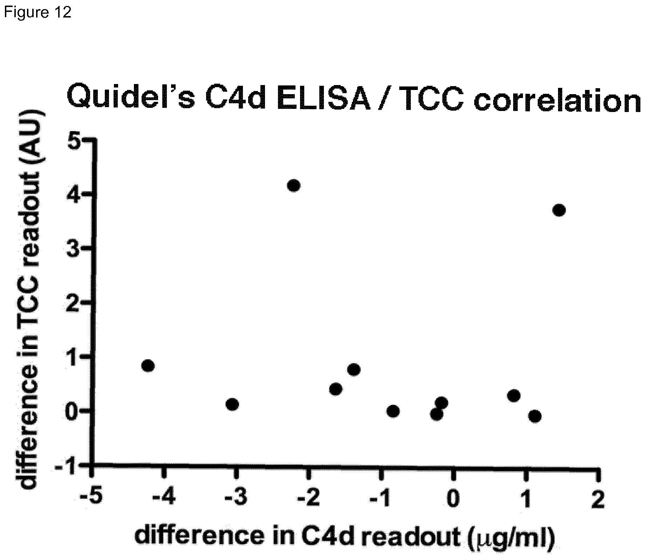

Figures 11 and 12 show the increase of C4d as measured in the two C4d ELISAs respectively, plotted against the results obtained with the TCC ELISA. We found that the data obtained in the C4d ELISA according to the invention correlated well with the TCC assay (Spearman r = 0,88, p = 0,0007, figure 11) whereas the C4d data obtained with the Quidel assay did not correlate with TCC data (Spearman r = -0,29, p = 0,38, figure 12).

We conclude that the antibodies and methods according to the invention are more suitable to detect C4d in biological samples than the prior art antibodies and methods.

Complement component C4d was recently described as a diagnostic and prognostic marker for a number of diseases, such as SLE, antibody-mediated graft rejection, rheumatoid arthritis, vasculitis, nephritis, antiphospholipid syndrome, scleroderma, Sjögrens syndrome, myositis, lung cancer and hematologic tumors.

Systemic lupus erythematosus is a chronic autoimmune disorder with complex etiology and multi-organ involvement. There are evidences that pathology of SLE is connected to defects in the complement system, as the scavenging function of complement is compromised (Pickering et al., 2000). On the other hand, self-antigens, which the body fails to dispose of during the course of SLE, retain complement activation potential thereby exacerbating disease (Leffler et al., 2012).

SLE is characterized by repeated periods of enhanced clinical manifestations termed flares and periods with lower disease intensity called remission. As there is no curative treatment for SLE, the aim of therapeutic intervention is to miminize the organ damage by counteracting the flare symptoms. An extensive array of biomarkers, including the measurement of total levels of complement C3 and C4 proteins is used to diagnose and monitor SLE, but none of them is reliable in predicting flares.

We therefore investigated whether the antibodies and methods according to the invention were able to predict flares of SLE and found that they indeed could predict and accurately determine whether an SLE patient experienced a flare of the disease. These experiments are described in Example 12.

The invention therefore relates to an in vitro method for monitoring the clinical status of an individual with Systemic Lupus Erythematosus wherein an antibody according to any one of claims 1 - 4 or 6 is contacted with a sample from the individual. Such a monitoring may include predicting or diagnosing a flare in disease activity.

As we have now established that the antibody according to the invention allows for a more accurate and specific assay for C4d, the antibody according to the invention may be advantageously used in a method for the diagnosis or prognosis of a disease selected from the group consisting of SLE, antibody-mediated graft rejection, rheumatoid arthritis, scleroderma, psoriatic arthritis, ankylosing spondylitis, vasculitis, nephritis, antiphospholipid syndrome, Sjögrens syndrome, myositis, lung cancer and hematologic tumors, wherein an antibody according to the invention is contacted with a sample from an individual and wherein it is concluded that the individual has an increased risk of having or developing said disease if the sample contains an elevated level of complement component C4d in comparison with a predetermined reference value. A skilled person is well aware of the various ways to obtain a reference value, for instance from a sample of a control subject.

The invention also provides a method for monitoring disease activity wherein the disease is characterized by flares of disease activity. Such disease may be selected from the group consisting of SLE, vasculitis and antibody-mediated graft rejection, wherein an antibody as described herein is contacted with a sample from an individual and wherein the level of C4d present in the sample is monitored over time.

The invention also provides a method for determining the efficacy of the treatment of a disease. In particular if the disease is selected from the group consisting of lung cancer, hematologic tumors, SLE, vasculitis and antibody-mediated graft rejection. Determining the efficacy of the treatment may be done in different ways. For instance, treatment for hematologic tumors makes use of complement as an effector mechanism, so that tumor cells are killed by complement. Therefore, clinical response to the drug is associated with complement activation and consequently higher C4d. In many other diseases, such as the ones listed above, elevated levels of C4d are a hallmark of pathological, spontaneous complement activation and treatment aims at elimination of pathogenic factor, which later on results in elimination of complement activation on this target. In such a method, the antibody as described herein is contacted with a sample taken from an individual before and after treatment and it is concluded that the individual responds to the treatment if the sample taken after treatment has a decreased or lower level of C4d in comparison to the C4d level in the sample taken before treatment.

### Legend to the figures

- Figure 1:: Processing of complement component C4b into C4c and C4d.
- Figure 2:: Reactivity of mouse monoclonal antibody A251 (Quidel) with immobilized complement components C4, C4b and C4d.
- Figure 3:: Reactivity of mouse monoclonal antibody LP69 (Abcam) with immobilized complement components C4, C4b and C4d.
- Figure 4:: Reactivity of rabbit monoclonal antibody A24-T (Abcam) with immobilized complement components C4, C4b and C4d.
- Figure 5:: Reactivity of rabbit monoclonal antibody SP91 (Abcam) with immobilized complement components C4, C4b and C4d.
- Figure 6:: Reactivity of serum from rabbit #3 immunized with a peptide comprising the C-terminal epitope SSTGR-COOH of C4d with immobilized complement components C4, C4b and C4d. Serum from rabbit #4 produced identical results and sera #3 and #4 were combined to form antibody BLOK C4d.
- Figure 7:: Graph showing the specific reaction of the BLOK C4d antibody with peptide NVTSSTGR-COOH contained in C4d and the absence of reactivity with peptide NVTSSTGRNGFK, which only occurs in C4b.
- Figure 8:: Graph showing the reactivity of the Quidel MicroVue™ C4d ELISA with increasing number of freeze/thaw cycles.
- Figure 9:: Graph showing the reactivity of the sandwich ELISA employing the BLOK C4d antibody with increasing number of freeze/thaw cycles.
- Figure 10:: Graph showing a comparison of the results in the Quidel MicroVue™ assay and a sandwich ELISA employing the BLOK C4d antibody wherein a low and high standard were tested (0 and 242 ng/ml respectively) and a normal human serum (NHS) and a heat inactivated normal human serum.
- Figure 11:: Graph showing the correlation of the BLOK C4d ELISA results with TCC ELISA results in 11 patients with leukemia treated with Rituximab™.
- Figure 12:: Graph showing the correlation of the Quidel MicroVue™C4d ELISA results with TCC ELISA results in 11 patients with leukemia treated with Rituximab™.
- Figure 13:: Graph showing the reactivity of BLOK C4d antibody with IgG-activated normal human serum in the presence of increasing amounts (0, 0,5, 1 and 2 microliter) of factor I-blocking antibody.
- Figure 14:: C4d levels in sera from patients with SLE taken in remission and in flares of the disease. Levels of C3 and C4 in the same sera are shown for comparison.
- Figure 15:: Comparison of C4d levels in sera from patients with SLE by pairing of flare-remission result from the same patient.
- Figure 16:: Reactivity of rabbit polyclonal antibody 12-5000 from American research products with immobilized complement components C4, C4b and C4d.
- Figure 17:: Reactivity of rabbit polyclonal antibody 58781 from Abcam with immobilized complement components C4, C4b and C4d.
- Figure 18:: Reactivity of rabbit polyclonal antibody 15376 catalogue number AP15376PU-S from Acris with immobilized complement components C4, C4b and C4d.
- Figure 19:: Reactivity of BLOKC4d antibody with selected patients sera.

### Examples

### Example 1: Preparation of C4d protein.

A solution of 200 microgram/ml C4b (Complement Technology Inc. 4801 Troup Hwy, Suite 701, Tyler, Texas 75703 USA, cat. no. A108, lot #5c) mixed with 20 microgram/ml plasma purified factor I [11] and 400 microgram/ml plasma purified C4BP [12] in a final volume of 50 microliter in Tris-buffered saline pH 8.0 was incubated at 37°C for 5 hours.

### Example 2: ELISA with C4 and its fragments C4b, and C4d

Microtiter plates (Maxisorp, Nunc) were coated overnight at room temperature (RT). Fifty microliters of a solution of 5 microgram/ml of C4 in PBS (Complement technology, cat. no. A105, lot #22a) or C4b (Complement Technology, cat. no. A108, lot#5c) or C4d preparation, obtained as described in example 1, was added to each well. The microtiter plate was then washed with wash buffer (50 mM Tris pH 8, 150 mM NaCl, 0.1% Tween 20), and blocked with 200 microliter of blocking buffer (1% BSA in wash buffer) for 2 hours at RT. Sera or purified antibodies, diluted 1:1000 in 50 microliter of blocking buffer were added for 1hour and incubated at RT, followed by washing and incubation with 50 microliter of goat anti rabbit-HRP antibody (Dako) diluted in the same blocking buffer. After final washing, 50 microliter of the standard OPD Tablets solution (Dako, prepared according to the manufacturer's instruction) was added to the wells and colorimetric reaction was stopped with 0.5 M H2SO4 once satisfactory color was obtained. Absorbance was measured at 490 nm in a microplate reader (Varian).

### Example 3: Immunization of rabbits

We immunized rabbits with peptides (two rabbits per peptide) corresponding to 5 amino acids from N-terminal and C-terminal sequences of C4d, which were elongated with a glycine / cysteine spacer. The sequence of the two peptides used for immunization was: N-terminal C4d part: NH2-TLEIPGGGC (SEQ ID NO: 1) and C-terminal C4d part: CGGSSTGR-COOH (SEQ ID NO: 2).

The peptides were synthesized using standard F-moc chemistry by Innovagen AB (Sweden). The peptides were then coupled to keyhole limpet hemocyanin. Immunization was performed according to standard protocols of Agrisera AB. In short, rabbits were immunized at t=0 with 200 micrograms of peptide in Freund's Complete Adjuvant (FCA) followed by booster injections of 200 microgram of peptide in Freund's Incomplete Adjuvant (FIA) at t=27 days, followed by two booster injections of 100 microgram of peptide in FIA at t=54 and t=81 days. Animals were bled and sacrificed at t=92 days.

The sera from the two rabbits immunized with the C-terminal peptide were similar in specificity and are referred to herein as BLOK C4d antibody.

### Example 4; Specificity of BLOK C4d antibody.

We established the specificity of BLOK C4d by determining its reactivity with samples wherein the formation of C4d was blocked by an antibody against factor I (mouse monoclonal antibody to human Factor I (#1) Quidel Corp., USA). Complement Factor I (fl) is a protein of the complement system, first isolated in 1966 in guinea pig serum [14] that regulates complement activation by cleaving cell-bound or fluid phase C3b and C4b [15]. When factor I is blocked, the formation of C4d from C4b is blocked.

Two microliter of normal human serum was mixed with 0, 0,5, 1 and 2 microliter of the factor I antibody (1,2 mg/ml) and incubated for 5 minutes on ice. Two microliters of heat-aggregated IgG (10 mg/ml) was added and mixed with 25 microliter of PBS containing 1 mM Ca and Mg and incubated for 45 minutes at 37 oC. The mixture was then diluted with 40 mM EDTA-GVB buffer up to 300 microliter. Fifty microliter of this preparation was used per well of the microELISA plate prepared according to example 6..

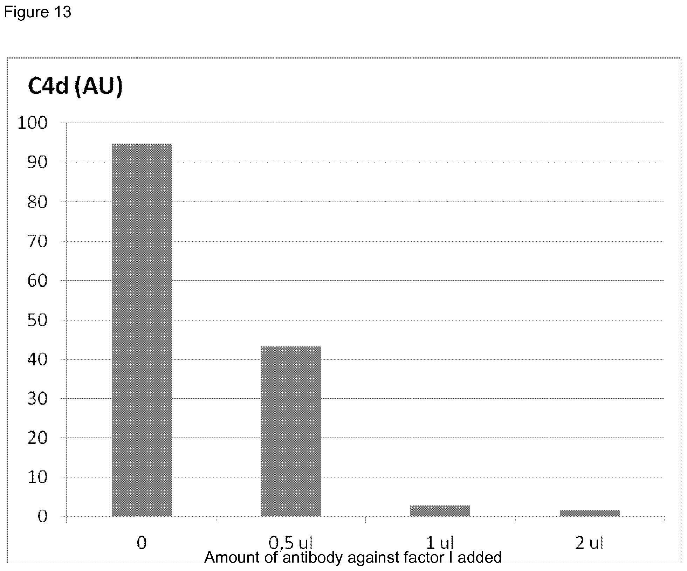

It was observed that the C4d level of the samples decreased with increasing amount of factor I blocking antibody (figure 13).

We concluded that the BLOK C4d antibody was specific for C4d since it did not show any reactivity with samples containing all components of the classical complement pathway, except C4d.

Control experiments performed with heat inactivated human serum showed no signal, whereas 100% signal was obtained in samples wherein the antibody against factor I was replaced by an equivalent amount of control antibody (MK54 against protein S).

### Example 5: Preparation of reagents

Heat inactivated normal human serum (NHS) was prepared by incubation of NHS at 56°C for 60 min in order to inactivate complement.

Heat-aggregated IgG was prepared by heating an IgG solution (Immuno, Austria) of 10 mg/ml in PBS at 63 °C for 30 minutes. After centrifugation at 15.000 x G for 5 minutes, the pellet was discarded.

A standard was prepared by mixing 2 microliters of NHS with 2 microliter of heat-aggregated IgG (10 mg/ml), diluted up to 25 microliter with PBS buffer containing 1 mM Ca and Mg and incubated for 1 h at 37°C. The mixture was then diluted up to 200 microliter with 40 mM EDTA-GVB (2.075 mM veronal buffer pH 7.3, 10 mM EGTA, 7 mM MgCI2, 0.083% gelatin, 116 mM glucose, 60 mM NaCl). Fifty microliter of such sample was loaded per ELISA well and defined as containing 1 arbitrary unit (AU) of C4d whereas serial dilutions of this standard completed the standard curve. Buffer only (40 mM EDTA-GVB) was considered as 0 value.

### Example 6: C4d capture assay

Maxisorp 96-well plates (Nunc) were coated overnight with 50 microliter of BLOK C4d antibody diluted 10 times in 0.1 M carbonate buffer pH 9.6 containing 0,02% sodium azide. The microtiter plate was blocked with 200 microliter blocking buffer (3% fish gelatine (Nordic) in wash buffer) for 1 h at 37 °C. After washing, 50 microliters of sample were loaded and incubated for 1 h at 37 °C. After washing, 50 microliter of detecting antibody (anti C4d mouse monoclonal, Quidel, #A213, diluted 1:1500 in blocking buffer) was applied followed by another washing cycle and 50 microliter of goat-anti mouse HRP (Dako) diluted 1:1000 in blocking buffer. Fifty microliter of the standard OPD Tablets solution (Dako, prepared according to the manufacturer's instruction) was added to the wells and colorimetric reaction was stopped with 0.5 M H₂SO₄ once satisfactory color was obtained. Absorbance was measured at 490 nm in microplate reader. Control experiments were performed with affinity purified antibodies as described in example 11, diluted until a final concentration of 3,5 microgram/ml IgG. Affinity purified antibodies gave identical results as the BLOK C4d antibody.

### Example 7: reactivity of commercial anti-C4d antibodies and BLOK C4d antibody.

Commercial antibodies used herein were A251 from Quidel , lot no. 910499, clone LP69 from Abcam, product no. ab167093, lot no. GR126509-6, clone A24-T from Abcam, product no. ab136921, lot no. GR161597-2, clone SP91 from Abcam, product no. ab183311, lot no. GR181800-1, rabbit polyclonal anti-C4d 12-5000 from American Research Products, lot no. 41200, rabbit polyclonal anti-C4d 58781 from Abcam, lot no. GR173176-3 and rabbit polyclonal anti C4d Ab CPAMD2 catalogue no. AP15376PU-S from Acris, lot no. 130618MC.

ELISA assays were prepared and performed as detailed in Example 2. All antibodies were tested at 10 microgram/ml, except for antibody SP91, which was diluted 1:50, and rabbit polyclonal anti C4d Ab anti-C4d 58781 from Abcam, which was prediluted by the manufacturer and was ready-to-use.

Antibodies (50 microliter), appropriately diluted in blocking buffer were incubated for 1 h at 37 °C followed by washing and 1 h incubation with 50 microliter of secondary antibody (goat-anti rabbit-HRP from Dako diluted 1:1000 for A24-T, SP91 and BLOK C4d or goat-anti mouse-HRP from Dako diluted 1:1000 for LP69 and A251, respectively). After final washing, 50 microliter of the standard OPD Tablets solution (Dako, prepared according to the manufacturer's instruction) was added to the wells and colorimetric reaction was stopped with 0.5 M H₂SO₄ once satisfactory color was obtained. Absorbance was measured at 490 nm in microplate reader. Final result was calculated by subtracting of the readout for negative control (measurement at 490 nm when no primary Ab was added). Results are shown in figures 2 - 6 and 16 - 18.

### Example 8: Peptide ELISA

An ELISA for the C-terminal C4d part was prepared by coating Maxisorp plates (Nunc) with 50 microliters of PBS containing 50 microgram/ml peptide with the sequence Cys-NVTLSSTGR-COOH (SEQ ID NO: 3) or Cys- NVTLSSTGRNGFK (SEQ ID NO: 4) at 37 °C for 1 h.

The microtiter plate was then washed with wash buffer (50 mM Tris pH 8, 150 mM NaCl, 0.1% Tween 20), and blocked with 200 microliter of blocking buffer (1% BSA in wash buffer) for 2 hours at RT. Antibodies (50 microliter) diluted in blocking buffer were incubated for 1 h at 37 °C followed by washing and 1 h incubation with 50 microliter of secondary antibody (goat-anti rabbit-HRP from Dako diluted 1:1000 for A24-T, SP91 and BLOK C4d or goat-anti mouse-HRP from Dako diluted 1:1000 for LP69 and A251, respectively). After final washing, 50 microliter of the standard OPD Tablets solution (Dako, prepared according to the manufacturer's instruction) was added to the wells and colorimetric reaction was stopped with 0.5 M H₂SO₄ once satisfactory color was obtained. Absorbance was measured at 490 nm in microplate reader. Final result was calculated by subtracting of the readout for negative control (measurement at 490 nm when no primary Ab was added).

### Example 9: Leukemia / lymphoma patients group.

Patients suffering from non-Hodgkin's lymphomas or chronic lymphocytic leukemia (CLL) were diagnosed at Karolinska University Hospital in Stockholm, Sweden and handled by prof. Anders Osterborg. From the patients qualified for rituximab treatment (as a single agent therapy or together with chemotherapy regimens) EDTA-plasma samples were collected immediately before and after the first infusion of the drug at standard therapeutic dose (375 mg/m²).

Some of the plasma samples were tested for the stability of C4d readout over the freeze-thawing cycles. For this purpose, they were aliquoted to 10 microliter and subsequently frozen at -80 °C and thawed at RT up to reaching liquid state. Freezing-thawing was repeated according to the desired number of cycles.

### Example 10: TCC assay

TCC assay was performed with mouse monoclonal anti C9-neoepitope antibody clone ae11 as catcher antibody and biotinylated anti-C6 monoclonal antibody clone 9C4 as detecting antibody, as previously described [13]. One hundred microliter of catcher antibody per well diluted in PBS containing 0,02% sodium azide was immobilized on Maxisorp plate (Nunc) at RT overnight. Detection of secondary antibody was performed with 100 microliter of streptavidin-HRP conjugate (R&D, USA) per well, thereafter the assay was developed with OPD tablets (Dako) and absorbance at 490 nm was measured.

### Example 11: affinity purification of BLOK C4d antibody

Notwithstanding the fact that the BLOK C4d antibody provided excellent results in terms of specificity and sensitivity, the antibody may be purified even further by affinity purification using peptides as disclosed herein. A particularly advantageous method was as follows.

Three ml of BLOK C4d antiserum from rabbit #3 was used to purify C4d-specific antibodies with affinity chromatography. Sulpholink beads (10 ml of beads, Thermo Scientific) were conjugated with 12 mg of the peptide according to SEQ ID NO: 3 (GenScript, USA) according to the manufacturer's protocol. The column was equilibrated with PBS buffer and the antiserum sample was loaded. After washing with PBS, the peptide-binding fraction was eluted with 0.1 M glycine pH 2.5 and immediately neutralized with 1/10 volume of 1M Tris pH 8.0. Neutralized fractions of the eluate were loaded onto another Sulpholink column conjugated with a peptide according to SEQ ID NO: 4 (GenScript, USA). This column was used for negative selection of antibodies, which potentially could bind C4d-C4c boundary epitopes (if any) and therefore not specific for C4d. The unbound fraction was collected, dialysed against PBS buffer and concentrated using 10 kDa cut-off device (Vivaspin, USA).

### Example 12: BLOK C4d antibody predicts and diagnoses flares of SLE.

Ninety-eight SLE patients fulfilling four or more American College of Rheumatology classification criteria (Tan et al., 1982), were included in the study. Plasma from the patients had been collected at the Department of Rheumatology in Lund, Sweden from 1984 to 2009. Samples from two time points were chosen: one time point when patients had their highest SLE disease activity index 2000 (SLEDAI-2K, Gladmann, D., et al., J. Rheumatol (2002) 29: 288-291), this was defined as "flare", and samples from another time point, when the patients had the lowest disease activity as evaluated by an experienced clinician, which was defined as "remission".

A data set with routine laboratory analyses (including measurements of C3 and C4) was available for all the samples. Plasma samples from 77 healthy donors were included as controls. C4d level was measured in patients and controls using the C4d ELISA based on BLOK antibody as described in Example 6. Dilutions of purified C4d from Quidel C4d MicroVue kit were used as a standard.

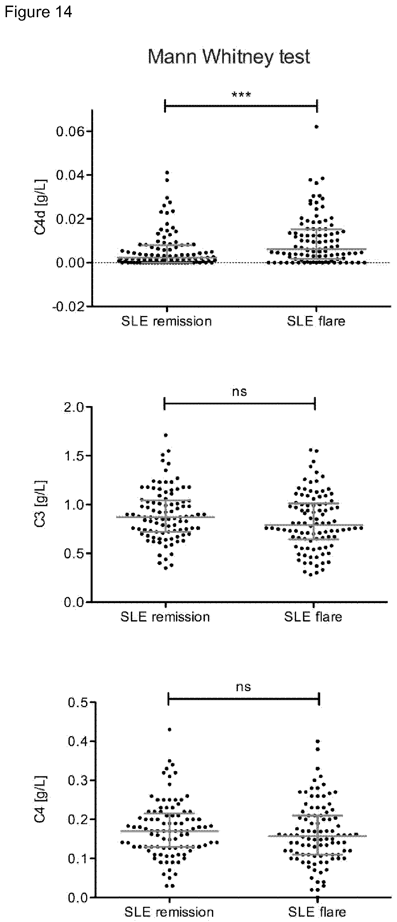

When all patients were taken into analysis, a strong significant difference could be detected between C4d levels in flare and remission (figure 14, p = 0.0005 by Mann Whitney test t, ***), in contrast to differences in total C3 and C4 levels (p = 0.063 and 0.2065, n.s.).

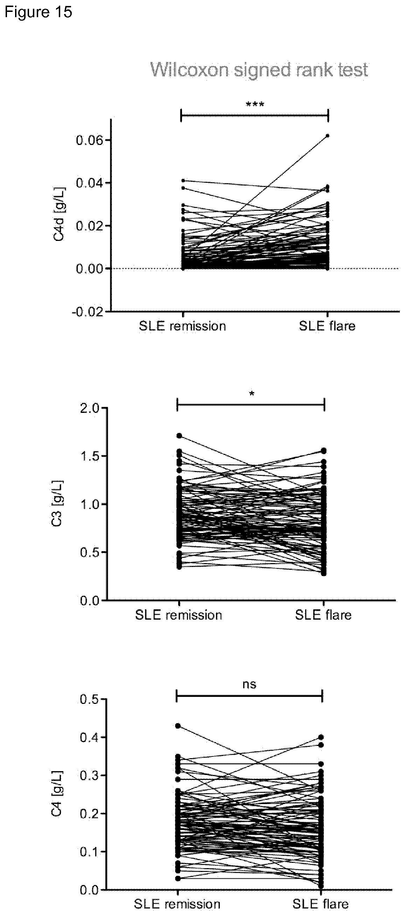

When Wilcoxon signed rank test (in which pairing of flare-remission result from the same patient was taken into account) was performed in the same data set, a strong significant difference could be detected between C4d levels in flare and remission (figure 15, p = <0.0001 ***). In contrast to that, C3 levels were slightly decreased during flare compared to remission and became significant at much lower level (p = 0.0275 *). C4 levels were not significantly different at all (p = 0.1085, n.s.).

We conclude that the C4d antibodies and methods according to the invention provide an improved assay, which was able to significantly (p < 0.05) distinguish SLE flares from remission either when analysis was performed for whole cohort or with pairing the results from individual patients.

### Example 13: BLOK C4d antibody distinguishes patients with rheumatoid arthritis, scleroderma, psoriatic arthritis and ankylosing spondilytis from healthy controls.

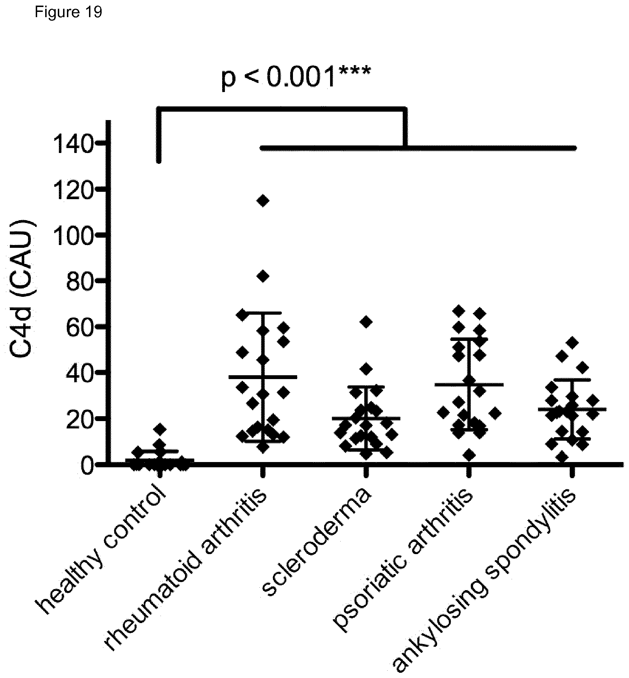

Samples were obtained from 20 individuals suffering from either rheumatoid arthritis, scleroderma, psoriatic arthritis or ankylosing spondylitis. A group of 20 healthy individuals served as controls. These sera were tested in the BLOK C4d ELISA as described herein. We found that the BLOK C4d antibody was able to distinguish the patient groups from the normal controls (Table 1 and figure 19).

**Table 1: Reactivity of BLOK C4d antibody with patients sera.**

| **GROUP** | **Group size N=** | **Mean** | **St. Dev** | **P value *** |
|---|---|---|---|---|
| Normal control | 20 | 1,87 | 3,96 | |
| Rheumatoid Arthritis | 20 | 38,05 | 28,02 | < 0,001 |
| Scleroderma | 20 | 20,10 | 13,73 | < 0,001 |
| Psoriatic arthritis | 20 | 34,86 | 19,72 | < 0,001 |
| Ankylosing spondylitis | 20 | 24,07 | 12,88 | < 0,001 |

| | | | | |
|---|---|---|---|---|
| * calculated for comparison to healthy (normal) control group, according to Kruskal-Wallis test with Dunn's multiple comparison post-test. | | | | |

### References

1. Janeway CAJ, Traves P, Walport M: Immunobiology: The Immune System in Health and Disease. 5th edition. The Immune System in Health and Disease 2001, New York.
2. Walport MJ: Complement. First of two parts. The New England journal of medicine 2001, 344:1058-1066.
3. Ajona D, Pajares MJ, Corrales L, Perez-Gracia JL, Agorreta J, Lozano MD, Torre W, Massion PP, de-Torres JP, Jantus-Lewintre E, et al: Investigation of complement activation product c4d as a diagnostic and prognostic biomarker for lung cancer. Journal of the National Cancer Institute 2013, 105:1385-1393.
4. Kalunian KC, Chatham WW, Massarotti EM, Reyes-Thomas J, Harris C, Furie RA, Chitkara P, Putterman C, Gross RL, Somers EC, et al: Measurement of cell-bound complement activation products enhances diagnostic performance in systemic lupus erythematosus. Arthritis and rheumatism 2012, 64:4040-4047.
5. Djamali A, Kaufman DB, Ellis TM, Zhong W, Matas A, Samaniego M: Diagnosis and management of antibody-mediated rejection: current status and novel approaches. American journal of transplantation : official journal of the American Society of Transplantation and the American Society of Transplant Surgeons 2014, 14:255-271.
6. Nuijens JH, Huijbregts CC, Eerenberg-Belmer AJ, Abbink JJ, Strack van Schijndel RJ, Felt-Bersma RJ, Thijs LG, Hack CE: Quantification of plasma factor XIIa-Cl(-)-inhibitor and kallikrein-Cl(-)-inhibitor complexes in sepsis. Blood 1988, 72:1841-1848.
7. Garred P, Mollnes TE, Lea T, Fischer E: Characterization of a monoclonal antibody MoAb bH6 reacting with a neoepitope of human C3 expressed on C3b, iC3b, and C3c. Scandinavian journal of immunology 1988, 27:319-327.
8. Mollnes TE, Lea T, Harboe M, Tschopp J: Monoclonal antibodies recognizing a neoantigen of poly(C9) detect the human terminal complement complex in tissue and plasma. Scandinavian journal of immunology 1985, 22:183-195.
9. Bergseth G, Ludviksen JK, Kirschfink M, Giclas PC, Nilsson B, Mollnes TE: An international serum standard for application in assays to detect human complement activation products. Molecular immunology 2013, 56:232-239.
10. Deppisch R, Schmitt V, Bommer J, Hansch GM, Ritz E, Rauterberg EW: Fluid phase generation of terminal complement complex as a novel index of bioincompatibility. Kidney international 1990, 37:696-706.
11. Blom AM, Kask L, Dahlbäck B: CCP1-4 of the C4b-binding protein a-chain are required for Factor I mediated cleavage of C3b. Mol Immunol 2003, 39:547-556.
12. Dahlbäck B: Purification of human C4b-binding protein and formation of its complex with vitamin K-dependent protein S. Biochem J 1983, 209:847-856.
13. Mollnes TE, Redl H, Hogasen K, Bengtsson A, Garred P, Speilberg L, Lea T, Oppermann M, Gotze O, Schlag G: Complement activation in septic baboons detected by neoepitope-specific assays for C3b/iC3b/C3c, C5a and the terminal C5b-9 complement complex (TCC). Clinical and experimental immunology 1993, 91:295-300.
14. Nelson R, Jensen J, Gigli I, Tamura N (1966). "Methods for the separation, purification and measurement of nine components of hemolytic complement in guinea-pig serum". Immunochemistry 3 (2): 111-35
15. Lachmann P, Müller-Eberhard H (1968). "The demonstration in human serum of "conglutinogen-activating factor" and its effect on the third component of complement". Journal of Immunology 100 (4): 691-8.
16. Leffler J., Martin M., Gullstrand B., Tyden H., Lood C., Truedsson L., Bengtsson A. A. and Blom A. M., 2012. Neutrophil extracellular traps that are not degraded in systemic lupus erythematosus activate complement exacerbating the disease. J Immunol 188, 3522-31.
17. Pickering M. C., Botto M., Taylor P. R., Lachmann P. J. and Walport M. J., 2000. Systemic lupus erythematosus, complement deficiency, and apoptosis. Advances in immunology 76, 227-324.
18. Tan E. M., Cohen A. S., Fries J. F., Masi A. T., McShane D. J., Rothfield N. F., Schaller J. G., Talal N. and Winchester R. J., 1982. The 1982 revised criteria for the classification of systemic lupus erythematosus. Arthritis and rheumatism 25, 1271-7.
19. Gladmann D.D., Ibanez D. and Urowitz M.B., Systemic lupus erythematosus disease activity index 2000. J. Rheumatol., 2002 29(2): 288-291.
20. Ali, S.A. et al., Frequent expression of C4d in hepatic graft-versus-host disease: Potential clue for diagnosis and distinguishing acute and chronic form, Transplant Immunology, 23: 77-80 (2010).

## Claims

1. Antibody specifically reactive with complement component C4d and not with complement component C4b or C4 wherein the antibody reacts with a peptide comprising an epitope consisting of amino acid sequence NVTLSSTGR-COOH and not with a peptide comprising an amino acid sequence NVTLSSTGRNGFK- COOH, **characterised in that** said antibody is capable of specifically binding to an epitope at the C-terminus of C4d comprising the amino acid sequence SSTGR-COOH.

2. Antibody according to claim 1, which is a monoclonal antibody.

3. Antibody according to claim 1, which is a polyclonal antibody.

4. Antibody according to any one of claims 1 - 3, which is a mouse, rat or a rabbit antibody.

5. Method for generating an antibody according to any one of claims 1 - 4 wherein a non-human animal is immunized with a peptide comprising the amino acid sequence SSTGR-COOH.

6. Antibody according to any of claims 1-4 obtainable by a method according to claim 5.

7. In vitro method for the detection of complement component C4d in a sample wherein the sample is contacted with an antibody according to any one of claims 1 - 4 or 6 and wherein binding between the antibody and C4d is detected.

8. Method according to claim 7 wherein the sample is a blood, serum, plasma, synovial fluid or liquor sample.

9. Method according to claim 7 or 8 wherein the sample is heat-inactivated.

10. Method according to any one of claims 7 - 9, which is selected from the group consisting of ELISA, RIA, EIA, and immunohistochemistry.

11. In vitro method for the diagnosis or prognosis of a disease selected from the group consisting of SLE, antibody-mediated graft rejection, rheumatoid arthritis, scleroderma, psoriatic arthritis, ankylosing spondylitis, vasculitis, nephritis, antiphospholipid syndrome, Sjogrens syndrome, myositis, lung cancer and hematologic tumors, wherein an antibody according to any one of claims 1 - 4 or 6 is contacted with a sample from an individual and wherein it is concluded that the individual has an increased risk of having or developing said disease if the sample contains an elevated level of complement component C4d in comparison with a predetermined reference value.

12. In vitro method for monitoring disease activity in an individual wherein the disease is selected from the group consisting of SLE, vasculitis and antibody-mediated graft rejection, wherein an antibody according to any one of claims 1 - 4 or 6 is contacted with a sample from the individual and wherein the level of C4d present in the sample is monitored over time.

13. In vitro method for determining the efficacy of the treatment of a disease in an individual, wherein the disease is selected from the group consisting of lung cancer, SLE, vasculitis and antibody-mediated graft rejection and wherein an antibody according to any one of claims 1 - 4 or 6 is contacted with a sample taken from an individual before and after treatment and wherein it is concluded that the individual responds to the treatment if the sample taken after treatment has a decreased or lower level of C4d in comparison to the C4d level in the sample taken before treatment.

14. Kit for the detection of complement component C4d, comprising an antibody according to any one of claims 1 - 4 or 6 immobilized on a solid support and a reagent for the detection of immune complexes.

15. In vitro method for predicting or diagnosing a flare in disease activity in an individual with systemic lupus erythematosus wherein an antibody according to any one of claims 1 - 4 or 6 is contacted with a sample from the individual.

## Patentansprüche

1. Antikörper, der spezifisch mit der Komplementkomponente C4d und nicht mit der Komplementkomponente C4b oder C4 reaktiv ist, wobei der Antikörper mit einem Peptid reagiert, das ein Epitop umfasst, das aus der Aminosäuresequenz NVTLSSTGR-COOH besteht und nicht mit einem Peptid, das eine Aminosäuresequenz NVTLSSTGRNGFK-COOH umfasst, **dadurch gekennzeichnet, dass** der Antikörper spezifisch an ein Epitop am C-Terminus von C4d anbinden kann, das die Aminosäuresequenz SSTGR-COOH umfasst.

2. Antikörper nach Anspruch 1, der ein monoklonaler Antikörper ist.

3. Antikörper nach Anspruch 1, der ein polyklonaler Antikörper ist.

4. Antikörper nach einem der Ansprüche 1 bis 3, der ein Maus-, Ratte- oder ein Kaninchen-Antikörper ist.

5. Verfahren zur Erzeugung eines Antikörpers nach einem der Ansprüche 1 bis 4, wobei ein nicht menschliches Tier mit einem Peptid immunisiert wird, das die Aminosäuresequenz SSTGR-COOH umfasst.

6. Antikörper nach einem der Ansprüche 1 bis 4, das durch ein Verfahren nach Anspruch 5 erlangt werden kann.

7. In-vitro-Verfahren zum Nachweis der Komplementkomponente C4d in einer Probe, wobei die Probe mit einem Antikörper nach einem der Ansprüche 1 bis 4 oder 6 in Kontakt gebracht wird, und wobei die Bindung zwischen dem Antikörper und C4d nachgewiesen wird.

8. Verfahren nach Anspruch 7, wobei die Probe eine Blut-, Serum-, Plasma-, Synovialflüssigkeits- oder Liquorprobe ist.

9. Verfahren nach Anspruch 7 oder 8, wobei die Probe hitzeinaktiviert wird.

10. Verfahren nach einem der Ansprüche 7 bis 9, das aus der Gruppe bestehend aus ELISA, RIA, EIA und Immunhistochemie ausgewählt ist.

11. In-vitro-Verfahren zur Diagnose und Prognose einer Erkrankung, die aus der Gruppe bestehend aus SLE, Antikörper-vermittelter Transplantatabstoßung, rheumatoider Arthritis, Sklerodermie, psoriatischer Arthritis, ankylosierender Spondylitis, Vaskulitis, Nephritis, Antiphospholipid-Syndrom, Sjögren-Syndrom, Myositis, Lungenkrebs und hämatologischen Tumoren ausgewählt wird, wobei ein Antikörper nach einem der Ansprüche 1 bis 4 oder 6 mit einer Probe von einem Individuum in Kontakt gebracht wird, und wobei gefolgert wird, dass das Individuum ein erhöhtes Risiko hat, diese Krankheit zu haben oder zu entwickeln, wenn die Probe einen erhöhten Gehalt der Komplementkomponente C4d im Vergleich mit einem vorbestimmten Referenzwert aufweist.

12. In-vitro-Verfahren zur Überwachung der Krankheitsaktivität in einem Individuum, wobei die Krankheit aus der Gruppe bestehend aus SLE, Vasculitis und Antikörper-vermittelter Transplantatabstoßung ausgewählt wird, wobei ein Antikörper nach einem der Ansprüche 1 bis 4 oder 6 mit einer Probe des Individuums in Kontakt gebracht wird, und wobei der in der Probe vorhandene C4d-Gehalt zeitlich überwacht wird.

13. In-vitro-Verfahren zur Bestimmung der Wirksamkeit der Behandlung einer Krankheit in einem Individuum, wobei die Krankheit aus der Gruppe bestehend aus Lungenkrebs, SLE, Vaskulitis und Antikörper-vermittelter Transplantatabstoßung ausgewählt wird, und wobei ein Antikörper nach einem der Ansprüche 1 bis 4 oder 6 mit einer Probe in Kontakt gebracht wird, die einem Individuum vor und nach der Behandlung entnommen wurde, und wobei gefolgert wird, dass das Individuum auf die Behandlung anspricht, wenn die nach der Behandlung entnommenen Probe einen verringerten oder niedrigeren C4d-Gehalt im Vergleich zum C4d-Gehalt in der vor der Behandlung entnommenen Probe aufweist.

14. Kit zum Nachweis der Komplementkomponente C4d, der einen auf einem festen Träger immobilisierten Antikörper nach einem der Ansprüche 1 bis 4 oder 6 und ein Reagenz für den Nachweis von Immunkomplexen umfasst.

15. In-vitro-Verfahren zum Vorhersagen oder Diagnostizieren eines Wiederaufflammens der Krankheitsaktivität in einem Individuum mit systemischem Lupus erythematodes, wobei ein Antikörper nach einem der Ansprüche 1 bis 4 oder 6 mit einer Probe des Individuums in Kontakt gebracht wird.

## Revendications

1. Anticorps spécifiquement réactif avec un composant complément C4d et non réactif avec un composant complément C4b ou C4, dans lequel l'anticorps réagit avec un peptide comprenant un épitope constitué d'une séquence d'acides aminés NVTLSSTGR-COOH et ne réagit pas avec un peptide comprenant une séquence d'acides aminés NVTLSSTGRNGFK- COOH, **caractérisé en ce que** ledit anticorps est capable de se lier spécifiquement à un épitope au niveau de la terminaison C de C4d comprenant la séquence d'acides aminés SSTGR-COOH.

2. Anticorps selon la revendication 1, qui est un anticorps monoclonal.

3. Anticorps selon la revendication 1, qui est un anticorps polyclonal.

4. Anticorps selon l'une quelconque des revendications 1 à 3, qui est un anticorps de souris, de rat ou de lapin.

5. Procédé pour générer un anticorps selon l'une quelconque des revendications 1 à 4, dans lequel un animal non humain est immunisé avec un peptide comprenant la séquence d'acides aminés SSTGR-COOH.

6. Anticorps selon l'une quelconque des revendications 1 à 4, pouvant être obtenu par un procédé selon la revendication 5.

7. Procédé in vitro pour la détection d'un composant complément C4d dans un échantillon, dans lequel l'échantillon est mis en contact avec un anticorps selon l'une quelconque des revendications 1 à 4 ou 6 et dans lequel une liaison entre l'anticorps et C4d est détectée.

8. Procédé selon la revendication 7, dans lequel l'échantillon est un échantillon de sang, de sérum, de plasma, de fluide ou liqueur synovial.

9. Procédé selon la revendication 7 ou 8, dans lequel l'échantillon est inactivé par la chaleur.

10. Procédé selon l'une quelconque des revendications 7 à 9, qui est choisi dans le groupe constitué d'ELISA, RIA, EIA et immunohistochimie.

11. Procédé in vitro pour le diagnostic ou le pronostic d'une maladie choisie dans le groupe constitué de lupus érythémateux disséminé, rejet de greffe médié par anticorps, polyarthrite rhumatoïde, sclérodermie, arthrite psoriasique, spondylarthrite ankylosante, vasculite, néphrite, syndrome antiphospholipide, syndrome de Sjögren, myosite, cancer du poumon et tumeurs hématologiques, dans lequel un anticorps selon l'une quelconque des revendications 1 à 4 ou 6 est mis en contact avec un échantillon provenant d'un sujet et dans lequel on conclut que le sujet présente un risque accru d'avoir ou de développer ladite maladie si l'échantillon contient un taux élevé de composant complément C4d par comparaison avec une valeur de référence prédéterminée.

12. Procédé in vitro pour la surveillance d'une activité de maladie chez un sujet, dans lequel la maladie est choisie dans le groupe constitué de lupus érythémateux disséminé, vasculite et rejet de greffe médié par anticorps, dans lequel un anticorps selon l'une quelconque des revendications 1 à 4 ou 6 est mis en contact avec un échantillon provenant du sujet et dans lequel le taux de C4d présent dans l'échantillon est surveillé au fil du temps.

13. Procédé in vitro pour déterminer l'efficacité du traitement d'une maladie chez un sujet, dans lequel la maladie est choisie dans le groupe constitué de cancer du poumon, lupus érythémateux disséminé, vasculite et rejet de greffe médié par anticorps et dans lequel un anticorps selon l'une quelconque des revendications 1 à 4 ou 6 est mis en contact avec un échantillon prélevé auprès d'un sujet avant et après traitement et dans lequel on conclut que le sujet répond au traitement si l'échantillon prélevé après traitement a un taux diminué ou plus bas de C4d par comparaison avec le taux de C4d dans l'échantillon prélevé avant traitement.

14. Trousse pour la détection de composant complément C4d, comprenant un anticorps selon l'une quelconque des revendications 1 à 4 ou 6 immobilisé sur un support solide et un réactif pour la détection de complexes immuns.

15. Procédé in vitro pour prédire ou diagnostiquer une poussée d'activité de maladie chez un sujet souffrant de lupus érythémateux disséminé, dans lequel un anticorps selon l'une quelconque des revendications 1 à 4 ou 6 est mis en contact avec un échantillon provenant du sujet.


### SEQUENCE LISTING

<110> Euro-Diagnostica AB
   <120> ANTIBODIES SPECIFIC FOR COMPLEMENT COMPONENT C4d AND USES THEREOF
   <130> 291 WO
   <160> 4
   <170> PatentIn version 3.5
<210> 1
   <211> 9
   <212> PRT
   <213> Homo Sapiens
   <400> 1
   Thr Leu Glu Ile Pro Gly Gly Gly Cys
   1               5
<210> 2
   <211> 8
   <212> PRT
   <213> Homo Sapiens
   <400> 2
   Cys Gly Gly Ser Ser Thr Gly Arg
   1               5
<210> 3
   <211> 9
   <212> PRT
   <213> Homo Sapiens
   <400> 3
   Asn Val Thr Leu Ser Ser Thr Gly Arg
   1               5
<210> 4
   <211> 13
   <212> PRT
   <213> Homo Sapiens
   <400> 4
   Asn Val Thr Leu Ser Ser Thr Gly Arg Asn Gly Phe Lys
   1               5                   10

### SEQUENCE LISTING

<110> Euro-Diagnostica AB
<120> ANTIBODIES SPECIFIC FOR COMPLEMENT COMPONENT C4d AND USES THEREOF
<130> 291 WO
<160> 4
<170> PatentIn version 3.5
<210> 1
   <211> 9
   <212> PRT
   <213> Homo Sapiens
<400> 1
   Thr Leu Glu Ile Pro Gly Gly Gly Cys
   1               5
<210> 2
   <211> 8
   <212> PRT
   <213> Homo Sapiens
<400> 2
   Cys Gly Gly Ser Ser Thr Gly Arg
   1               5
<210> 3
   <211> 9
   <212> PRT
   <213> Homo Sapiens
<400> 3
   Asn Val Thr Leu Ser Ser Thr Gly Arg
   1               5
<210> 4
   <211> 13
   <212> PRT
   <213> Homo Sapiens
<400> 4
   Asn Val Thr Leu Ser Ser Thr Gly Arg Asn Gly Phe Lys
   1               5                   10